# EUROPEAN PATENT SPECIFICATION

(11) **EP 3 702 760 B1**
(45) Date of publication and mention of the grant of the patent: **24.06.2026**
(21) Application number: 18871181.6
(22) Date of filing: 23.10.2018
(51) Int. Cl.: G01J 3/443, G01N 21/67, G01N 33/00, G01J 3/02, G01J 3/18, G01J 3/28, G01M 3/40

(54) **GAS ANALYZER**
GASANALYSATOR
ANALYSEUR DE GAZ

(30) Priority: 24.10.2017 JP 2017205223
(43) Date of publication of application: 02.09.2020
(73) Proprietor: Marunaka Co., Ltd., Toyoake-shi, Aichi 470-1141 (JP)
(72) Inventor: NAKAGAWA Mitsugu, Toyoake-shi, Aichi 470-1141 (JP); ENOMOTO Kazumi, Toyoake-shi, Aichi 470-1141 (JP); YAMAMOTO Setsuo, Ube-shi, Yamaguchi 755-8611 (JP); KURISU Hiroki, Ube-shi, Yamaguchi 755-8611 (JP)
(74) Representative: Becker, Eberhard
(86) International application number: PCT/JP2018/039358
(87) International publication number: WO 2019/082893

(56) References cited:
- WO-A1-2012/005199
- WO-A1-2016/141463
- WO-A1-2016/141463
- CA-A- 1 141 812
- JP-A- 2012 517 586
- JP-A- 2016 170 072
- JP-A- 2016 170 072
- JP-A- 2017 072 491
- JP-A- 2017 072 491
- JP-A- H03 167 450
- JP-B2- 2 926 943
- JP-B2- 5 415 420
- US-A- 2 670 649
- US-A- 4 147 431
- US-A- 5 541 519
- US-A- 6 133 740
- BROEKAERT J A C: "GLOW DISCHARGE ATOMIC SPECTROSCOPY", APPLIED SPECTROSCOPY, THE SOCIETY FOR APPLIED SPECTROSCOPY. BALTIMORE, US, vol. 49, no. 7, 1 July 1995 (1995-07-01), pages 12A - 19A, XP000514902, ISSN: 0003-7028, DOI: 10.1366/0003702953964732

## Description

### [Technical Field]

The present invention relates to a gas analyzer and more particularly relates to a gas analyzer that uses discharge optical emission having a low lower limit of detection.

### [Background Techniques]

It is effective for conducting process management in various vacuum equipment including a vacuum equipment for manufacturing electronic devices to perform gas analyzing in vacuum process. In the explanation below, extents of vacuum will be referred to as a medium vacuum for 10⁻¹ Pa to 10² Pa, a high vacuum for 10⁻⁵ Pa to 10⁻¹ Pa and an ultrahigh vacuum for 10⁻⁹ Pa to 10⁻⁵ Pa, according to Japanese Industrial Standards (JIS).

Gas analyzers that can be applied to various vacuum equipment require following matters:
(1) to have a wide operable range from an ultrahigh vacuum of 10⁻⁷ Pa to a medium vacuum of 10² Pa, and
(2) to have a low lower limit of detection of partial pressure with an ultralow concentration of 10⁻⁷ Pa. With a quadrupole mass spectrometer that can detect a tiny amount of gas concentration, among conventional gas analyzers, a hot-filament wears in a vacuum over a medium vacuum of 1 Pa and carry distance of ionized gas for analyzing mass is long as of several centimeters or more, so that ionized gas collides with other gas, making it difficult to classify gasses for each mass number. Hence, operation in a medium vacuum is difficult in principle.

In general, discharge generated by applying a high voltage between a cathode and an anode confronted with each other in a vacuum occurs in a medium vacuum and has a lower limit of pressure of about 1 Pa. For maintaining discharge in a high vacuum range below it, modules that maintain discharge by applying a high voltage electric field and a magnetic field are utilized for a cold cathode ionization gauge, an ion pump or the like. Such gas analyzers detecting optical emission of excited gas with discharge generated by applying a high voltage electric field and a magnetic field (referred to as magnetic discharge below) are seen to be useful as gas analyzers that can be operated in a wide vacuum range from a high vacuum to a medium vacuum and a number of prior techniques have been proposed.

Patent Document 1 discloses a gas analyzer used for a vacuum equipment that employs a Penning discharge method as one of magnetic field discharge methods for a method of exciting gas and is equipped with a total pressure measurement mechanism by measuring ion current and a partial pressure measurement mechanism by measuring intensity of optical emission of various gases. In a case of Penning discharge method, however, a high voltage electric field is not perpendicular to a magnetic field, so that it is difficult to maintain steady discharge in a vacuum below 10⁻⁴ Pa and discharge generates weak optical emission. Due to this, a lower limit of partial pressure detection by measuring optical emission is about 10⁻³ Pa, which exhibits a problem in attaining a lower limit of partial pressure detection as of an ultralow concentration of 10⁻⁷ Pa.

Patent Document 2 and Patent Document 3 disclose gas analyzers that employ an inverted magnetron discharge method as one of magnetic field discharge methods for exciting gas and are equipped with a total pressure measurement mechanism by measuring ion current and a partial pressure measurement mechanism by measuring intensity of optical emission of various gases. A structure of a gas analyzer detecting optical emission by magnetic field discharge using an inverted magnetron discharge method will be explained referring to Fig. 9, after which Patent Document 2 and Patent Document 3 as prior arts will be explained below.

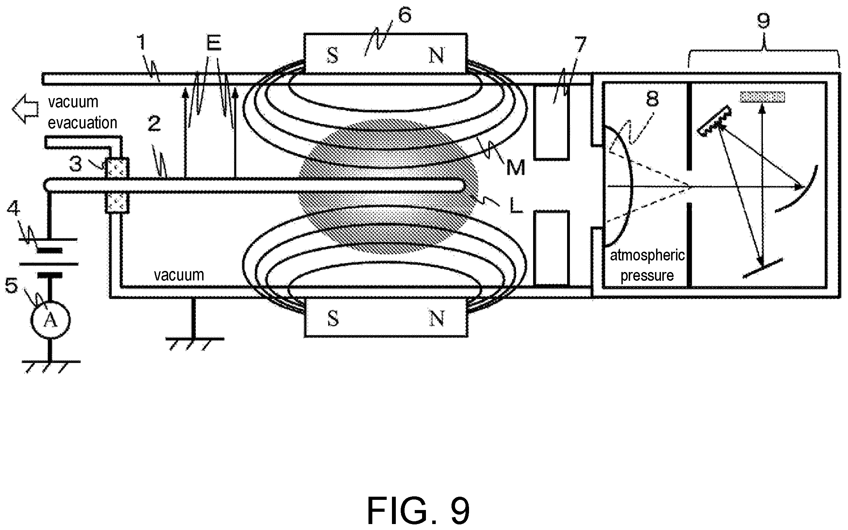

Fig. 9 shows a schematic view of a gas analyzer that detects optical emission by magnetic field discharge using an inverted magnetron discharge method. An electrode 2 as an anode is attached to a vacuum casing 1 as a cathode via an insulated terminal 3. A direct current source 4 and an ammeter 5 are connected with the electrode 2. Further, magnetic field applying module (a magnet) 6 is disposed outside of the vacuum casing 1.

Electric field E is created between the electrode 2 as an anode and the vacuum casing 1 as a cathode when direct current voltage of several kV is applied to the electrode 2. On the other hand, magnetic field M is created with magnetic field applying module 6. Electrons emitted from the vacuum casing 1 of a cathode are accelerated with the electric field E as well as subjected to a Lorentz force with an electric field E and a magnetic field M, thus making a spiral movement so as to coil around the magnetic field M. Hence, carry distance of electrons in a vacuum space becomes long and electrons are localized in the magnetic field M. Due to collision of the electrons with gas, gas is excited to be ionized or radicalized, thus creating discharge. As seen in Fig. 9, intensive discharge is created in the vicinity of sites where the electric field E is perpendicular to the magnetic field M, emitting light of discharge optical emission L by excited gas. Because of long carry distance of electrons and localization of electrons, it is possible to maintain discharge even in an ultrahigh vacuum of 10⁻⁷ Pa.

The light of discharge optical emission L by the excited gas passes a plate 7 having a hole and is focused on a light receiving element as light detecting module 9 using a focusing lens 8 so that light is detected. Here, cation gas excited by discharge collides with the vacuum casing 1 as a cathode, generating a sputter deposition phenomenon knocking out particles (atoms or molecules) from the material of the vacuum casing 1 and causing scattered particles to be attached to the insides of the vacuum casing. The plate 7 having a hole is placed for preventing a focusing lens 8 from getting dirt due to the scattered particles by the sputter deposition phenomenon. While the light of discharge optical emission L consists of optical emission of atoms or molecules for each gas species, wave length of the light is various according to gas species. It is possible to measure intrinsic optical mission of multitudes of gas species in a time shorter than several seconds by measuring light of discharge optical emission L using a multichannel spectrometer that can measure light of multiple wave lengths simultaneously as light detecting module 9. Intensity of intrinsic optical emission and pressure (partial pressure) of gas are measured preliminarily for each gas species and the intensity of optical emission is converted to a pressure of gas.

With magnetic field discharge by a prior art inverted magnetron method shown in Fig. 9, areas where magnetic field M by the magnet as magnetic field applying module 6 is parallel to an electrode axis of the electrode 2 (perpendicular to the electric field E) are distributed in the area where the magnet is disposed, due to which discharge is distributed in the direction of electrode axis. When this discharge optical emission is to be focused through a focusing lens 8, focusing positions extend to a wide range because light of discharge optical emission L is distributed widely in a direction of the electrode axis, so that only a part of the discharge optical emission is focused on the light detecting module 9 through the focusing lens 8. Due to this, although discharge can be maintained in an ultrahigh vacuum of 10⁻⁷ Pa with a prior art gas analyzer, a lower limit of a partial pressure detection by measuring optical emission with magnetic field discharge is about 10⁻⁵ Pa.

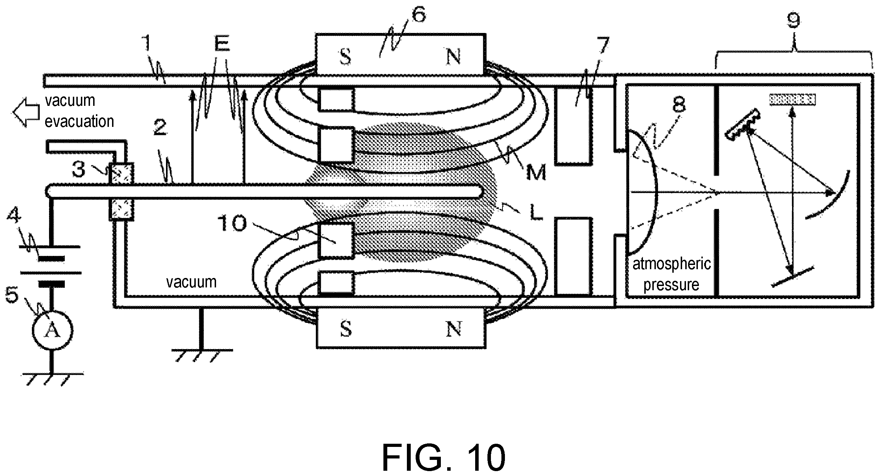

With a gas analyzer using discharge optical emission with magnetic field discharge of Patent Document 2 shown in a schematic view of Fig. 10, a metal plate 10 having holes connected conductively to the vacuum casing 1 as a cathode is placed in the gas analyzer shown in Fig. 9, so that electric field E in the central hole of the metal plate 10 having holes is intensified to generate intensive light of discharge optical emission. Due to this, it is possible to detect gas of 10⁻⁶ Pa. However, it is not clear with this gas analyzer whether intensive optical emission can be actually created at the central hole of the metal plate 10 having a central hole as explained.

While a central hole of a cathode disc has a diameter of several mm in Patent Document 2 ([0041]), the metal plate serves as an earth shield in this case, so that discharge is not generated inside of the hole. Actually, it is also described in [0047] that sputter deposition is not generated inside of the hole and optical emission is somewhat lowered. Even if intensive optical emission can be obtained inside of the central hole of the metal plate having holes, there is a problem in focusing optical emission in the central hole of the metal plate 10 having holes on the light detecting module 9 with a high efficiency and raising detection accuracy, because the focusing lens 8 is disposed far from the optical emission (described as about 10 cm) and a plate having a hole 7 for preventing the lens from getting dirt is placed in front of the lens.

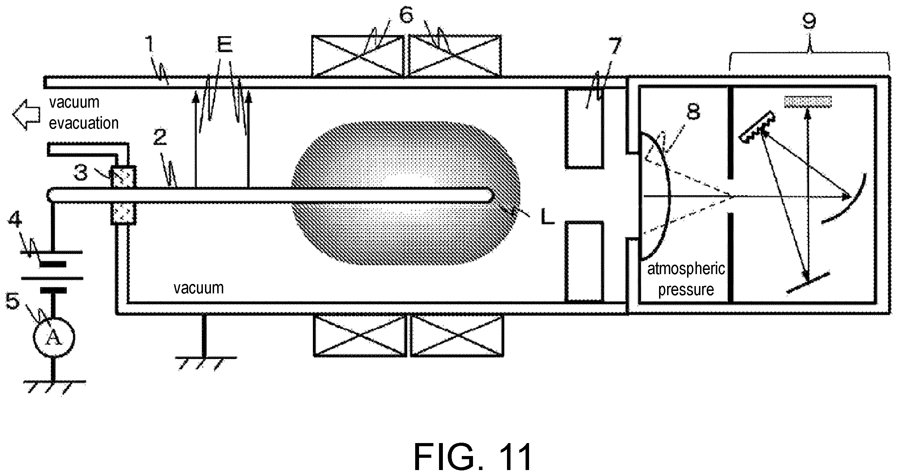

A gas analyzer using discharge optical emission with magnetic field of Patent Document 3 shown schematically in Fig. 11 is composed so that discharge optical emission is generated in a magnetic field with magnetic applying module 6 consisting of two cylindrical magnets in a gas analyzer of an inverted magnetron type, thus enabling to maintain discharge up to a vacuum of 10⁻⁷ Pa. However, increase of magnetic field M is not so high with intensity of discharge being similar as shown in Fig. 9. Consequently, a lower limit of partial pressure detection with measurement of discharge optical emission according to Patent Document 3 is considered to be about 10⁻⁵ Pa.

As explained above, although discharge can be maintained in an ultrahigh vacuum of 10⁻⁷ Pa with gas analyzers using magnetic field discharge by an inverted magnetron method of Patent Document 2 and Patent Document 3 as prior arts, a lower limit of detection of partial pressure with an ultralow concentration of 10⁻⁷ Pa has not been attained. It is considered to set a high voltage for discharge in order to solve this. However, this is not a practical way, because high sputter deposition is generated in this case and detection accuracy is lowered due to dirt of a focusing lens. On the other hand, it is also considered to employ a photon counting method enabling detection of extremely weak light for light detecting module. However, also this case is not a practical way because measurement of such extremely weak light requires a long time more than several minutes and equipment therefor is of a high cost.

### [Prior Art Documents]

### [Patent Documents]

[Patent Document 1]
   JP Published Patent Application No. S52-131780
[Patent Document 2]
   JP Patent No. 5,415,420
[Paten Document 3]
   JP Published Patent Application No. 2016-170072
JP 2017 072491 A discloses a leak inspection device and method, wherein a test body is disposed inside a test body vacuum chamber, the test body vacuum chamber is evacuated using an evacuating means in a state where the test body vacuum chamber is tightly sealed, a probe fluid is supplied under pressure to the test body from a probe fluid pressurizing/recovering system, and leak inspection is performed by measuring the partial pressure of the probe fluid leaking from the test body using a gas detection means. As the gas detection means, a highly durable gas detection means that can operate in a range from an ultrahigh vacuum to a medium vacuum is used.

### [Summary of the Invention]

### [Problems to be Solved by the Invention]

With vacuum equipment including a vacuum equipment for manufacturing electronic devices, it is effective to perform gas analyzing in a vacuum process for process managing. For a gas analyzer that can be applied to various vacuum equipment, following matters are required. That is,
(1) to have a wide operable range from an ultrahigh vacuum of 10⁻⁷ Pa to a medium vacuum of 10² Pa, and
(2) to have a low lower limit of detection of partial pressure with an ultralow concentration of 10⁻⁷ Pa.

However, while a quadrupole mass spectrometer among conventional gas analyzers attains a low limit of detection of the above (2), it cannot attain in principle a wide operable range of the above (1). Under such background, gas analyzers detecting discharge optical emission in process gas or residual gas in a vacuum have been proposed. However, although a wide operable range of the above (1) is attained to some extent, current situation of a low lower limit of detection of the above (2) is of 10⁻⁷ Pa, thus requiring a significant improvement. It is an object of the present invention to provide a gas analyzer that enables a high defection accuracy with a lower limit of detection in an ultrahigh vacuum of 10⁻⁷.

### [Means for Solving the Problems]

The present invention is provided by the appended claims. The following disclosure serves a better understanding of the present invention. The invention is created to solve the aforementioned problems.

The current invention provides a gas analyzer wherein the vacuum casing is formed to have a generally pipe shape as well as to be made of a conductive material so as to serve as the cathode electrode, the anodic electrode is made up of an elongated conductive member fixed to the vacuum casing on one end face side thereof so as to extend in a longitudinal direction of the vacuum casing within the vacuum casing as a cantilever and at least a tip side portion thereof is formed of a conductive ferromagnetic material, and a magnet as magnetic field applying module is placed in an area of discharge optical emission between the anodic electrode and an inner peripheral portion of the vacuum casing serving as the cathode electrode so as to concentrate magnetic field, so that discharge optical emission is localized in a vicinity of a tip of the anodic electrode.

According to an embodiment, a non-magnetic member is fixed to the vacuum casing on one end face side thereof so as to extend in a longitudinal direction within the vacuum casing as a cantilever, an elongated ferromagnetic member extending near to and along the anodic electrode as a cantilever is also fixed to the vacuum casing on the one end face side thereof so as to cause a tip thereof to be at an equivalent position to the tip of the anodic electrode, and a magnet as magnetic field applying module is placed in an area of discharge optical emission between the anodic electrode and an inner peripheral portion of the vacuum casing serving as the cathode electrode so as to concentrate magnetic field, so that discharge optical emission is localized in a vicinity of a tip of the anodic electrode.

According to the current invention, the magnet as the magnetic field applying module is provided on a peripheral face of the vacuum casing surrounding a position of the tip of the anodic electrode, so that discharge optical emission is localized in a vicinity of the tip of the anodic electrode.

According to an embodiment, the anodic electrode is fixed to the vacuum casing on one end face side thereof and, along therewith, a bar magnet as a bar magnetic field applying module is fixed to the vacuum casing on the other end face side so as to extend as a cantilever in a direction of extension of the anodic electrode, and a tip of the anodic electrode of the ferromagnetic material and one pole of the bar magnet are in an opposed positional relation with each other to concentrate magnetic field in a vicinity of the tip of the anodic electrode, so that discharge optical emission is localized in a vicinity of the tip of the anodic electrode.

According to an embodiment, the vacuum casing is formed of a conductive, ferromagnetic and soft magnetic material so as to serve as a cathode electrode, the anodic electrode consists of an elongated conductive member fixed to the vacuum casing on one end side thereof so as to extend as a cantilever in a longitudinal direction within the vacuum casing as well as a tip portion of the anodic electrode is formed of a conductive and ferromagnetic material, and a magnet as magnetic field applying module is placed so as to concentrate magnetic field in an area of discharge optical emission between the anodic electrode and an inner peripheral portion of the vacuum casing serving as a cathode electrode, so that most of magnetic flux from the magnet as the magnetic field applying module passes within the vacuum casing of a ferromagnetic and soft magnetic material for magnetic flux to be permeable well to concentrate magnetic field in a vicinity of the tip of the anodic electrode and localize discharge optical emission.

According to an embodiment, the vacuum casing has a generally pipe shape and is formed of a conductive, ferromagnetic and soft magnetic material so as to serve as a cathode electrode, the anodic electrode consists of an elongated conductive and non-magnetic member fixed to the vacuum casing on one end side thereof so as to extend as a cantilever in a longitudinal direction within the vacuum casing, an elongated ferromagnetic member extending near to and along the anodic electrode as a cantilever is also fixed to the vacuum casing on the one end face side thereof so as to cause a tip thereof to be at an equivalent position to the tip of the anodic electrode, and a magnet as magnetic field applying module is placed so as to concentrate magnetic field in an area of discharge optical emission between the anodic electrode and an inner peripheral portion of the vacuum casing serving as a cathode electrode, so that most of magnetic flux from the magnet as the magnetic field applying module passes within the vacuum casing of a ferromagnetic and soft magnetic material for magnetic flux to be permeable well to concentrate magnetic field in a vicinity of the tip of the anodic electrode and localize discharge optical emission.

According to an embodiment, a magnet as the magnetic field applying module is provided on a peripheral face of the vacuum casing surrounding a position of the tip of the anodic electrode, so that discharge optical emission is localized in a vicinity of the tip of the anodic electrode.

According to an embodiment, the anodic electrode is fixed to the vacuum casing on one end face side thereof and, along therewith, a bar magnet as a bar magnetic field applying module is fixed to the vacuum casing on the other end face side so as to extend as a cantilever in a direction of extension of the anodic electrode, and a tip of the anodic electrode of the ferromagnetic material and one pole of the bar magnet are in an opposed positional relation with each other to concentrate magnetic field in a vicinity of the tip of the anodic electrode, so that discharge optical emission is localized in a vicinity of the tip of the anodic electrode.

According to an embodiment, the vacuum casing is formed of a ferromagnetic and soft magnetic material, an elongated ferromagnetic member is fixed to the vacuum casing on one end side thereof so as to extend as a cantilever in a longitudinal direction within the vacuum casing, a bar magnet as a bar magnetic field applying module is fixed to the casing on the other end face side so as to extend as a cantilever in a direction of extension of the anodic electrode, a tip of the ferromagnetic member and one pole of the bar magnet are in an opposed positional relation with each other, and an anodic electrode and a cathode electrode are provided respectively in a manner to be opposed to each other with the tip of the elongated ferromagnetic member disposed between the electrodes, so that most of magnetic flux from the bar magnet as the bar magnetic field applying module passes within the vacuum casing of a ferromagnetic and soft magnetic material for magnetic flux to be permeable well to concentrate magnetic field in a vicinity of the tip of the anodic electrode and localize discharge optical emission.

According to an embodiment, discharge optical emission is enhanced and a low lower limit of detection can be attained by introducing a tiny amount of gas, other than one to be detected, that has a metastable excitation energy higher than an excitation energy for discharge optical emission of gas to be detected into the tightly closable vacuum casing in gas analyzing.

According to an embodiment, the tiny amount of gas, other than one to be detected, that has a metastable excitation energy higher than an excitation energy for discharge optical emission of gas to be detected is helium gas, nitrogen gas, air gas or argon gas.

### [Advantageous Effect of the Invention]

In a gas analyzer using discharge optical emission according to the present invention, in which a magnetic field is caused to be generated in a direction crossing an electric field generated when a high voltage is applied between electrodes and discharge optical emission generated in the magnetic field is detected,
(1) an anodic electrode is formed of a ferromagnetic material or a ferromagnetic material/materials is/are placed in a vicinity of the anodic electrode, and
(2) a vacuum casing is formed of a ferromagnetic and soft magnetic material so that a magnetic circuit for magnetic flux to be permeable is composed, thus intensifying a magnetic field generated between the magnet as magnetic field applying module and the ferromagnetic anodic electrode.

Due to this, discharge optical emission in the magnetic field is localized, thus increasing intensity of optical emission and, along with this, detection accuracy can be raised so that a lower limit of detection in an ultrahigh vacuum of 10⁻⁷ Pa can be secured.

Further, in a gas analyzer using discharge optical emission according to the present invention, in which a magnetic field is caused to be generated in a direction crossing an electric field generated when a high voltage is applied between electrodes and discharge optical emission is detected, a tiny amount of gas other than one to be detected is introduced, so that intensity of optical emission of the gas to be detected is increased as well as detection accuracy can be raised.

### [Brief Description of Drawings]

Fig. 1 is a view showing a composition of a gas analyzer according to an example outside the scope of the current invention.
Fig. 2 is a view showing a composition of a gas analyzer according to Embodiment 2 of the present invention.
Fig. 3 is a view showing a composition of a gas analyzer according to Embodiment 3 of the present invention.
Fig. 4 is a view showing a composition of a gas analyzer according to Embodiment 4 of the present invention.
Fig. 5 is a view showing a composition of a gas analyzer according to Embodiment 5 of the present invention.
Fig. 6 is a view showing another arrangement of an anodic electrode in the gas analyzer according to Embodiments 1 to 4.
Fig. 7 is a view showing still another arrangement of an anodic electrode in the gas analyzer according to Embodiments 1 to 4.
Fig. 8 is a schematic view showing a composition of performance measuring apparatus of a gas analyzer according to the present invention.
Fig. 9 is a view showing a composition of a conventional gas analyzer using discharge optical emission.
Fig. 10 is a view showing a composition of a conventional gas analyzer using discharge optical emission.
Fig. 11 is a view showing a composition of a conventional gas analyzer using discharge optical emission.

### [Detailed Description of Preferred Embodiments]

While a gas analyzer using discharge optical emission according to the present invention is similar to one by a conventional case shown in Figs. 9 to 11, in providing an anodic electrode within a tightly closable casing serving as a cathode electrode, providing magnetic field applying module generating a magnetic field crossing a direction of an electric field and analyzing gas by detecting discharge optical emission created in the magnetic field with a high voltage applied to the electrodes, the gas analyzer further provides following means in order to attain a lower limit of detection in an order of 10⁻⁷ Pa.

### (1) New creating means of discharge optical emission for localizing discharge optical emission

### (a) Placing a ferromagnetic material in an area of discharge optical emission (Embodiments 1 to 5)

An anodic electrode is formed of a ferromagnetic material or a ferromagnetic material/materials is/are placed in a vicinity of the anodic electrode, so that discharge optical emission in a magnetic field is localized in a vicinity of a tip of an anodic electrode to increase intensity of optical emission and raise detection accuracy.

### (b) Composing a magnetic circuit for magnetic flux to be permeable (Embodiments 3 to 5)

A vacuum casing is formed of a ferromagnetic and soft magnetic material so that a magnetic circuit for magnetic flux to be permeable is composed, thus intensifying a magnetic field generated between the magnet as magnetic field applying module and the ferromagnetic anodic electrode. Due to this, discharge optical emission in the magnetic field is further localized, intensity of optical emission is increased and, along with this, detection accuracy is raised.

While discharge optical emission is localized so that detection accuracy is raised, this is due to a situation such that an area of intensive optical emission on an optical emission detecting portion in a direction of optical axis of a focusing lens is narrowed, so that the area of optical emission is concentrated effectively in a vicinity of focusing point of a focusing lens, allowing more optical emission to be detected.

### (2) Introduction of a tiny amount of gas

A tiny amount of gas other than one to be detected is introduced. Due to this, when ionized gas or radical gas having a long life collides with gas to be detected, the gas to be detected is excited to create optical emission, thus increasing discharge optical emission of gas to be detected.

Embodiments of the present invention will be explained below. Manners of using gas analyzers according to the Embodiments will be explained relating to performance of the gas analyzers.

### [Example 1]

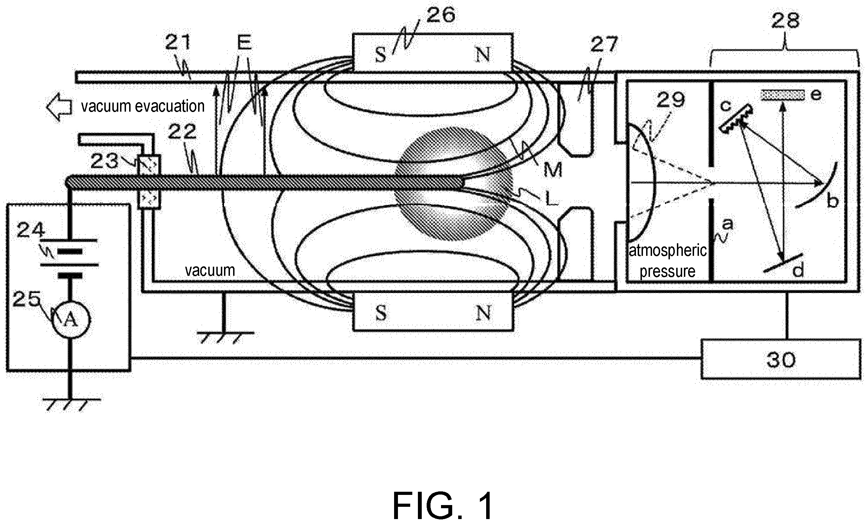

A gas analyzer according to Example 1, outside the scope of the present invention is shown in Fig. 1. Fig. 1 shows a composition of a gas analyzer according to Example 1, in which an elongated ferromagnetic electrode as an anode 22 is provided within a tightly closable pipe-shaped vacuum casing 21 to extend in a longitudinal direction of the casing, and a pipe-shaped magnet as magnetic field applying module 26 is placed on an outer periphery of the vacuum casing 21. The vacuum casing 21, formed of a conductive material, serves as a cathode electrode, the ferromagnetic anodic electrode 22 is attached the cylindrical vacuum casing 21 at one end side thereof via an insulating member 23 so as to extend in a longitudinal direction of the vacuum casing 21 as a cantilever beam and the other end of anodic electrode 22, protruding out of the vacuum casing 21, is connected to a direct current power source 24. 25 denotes an ammeter.

The anodic electrode 22 extends up to some distance before the other end side of the pipe-shaped vacuum casing 21 as a cantilever beam within the vacuum casing and a magnet as magnetic field applying module 26 is provided on an outer periphery of the vacuum casing 21 at a position surrounding the tip portion of the anodic electrode 22. The magnet may be disposed so as to surround the pipe-shaped vacuum casing 21 or may be disposed in such a manner that two magnets are disposed in opposing positions with the tip portion of the anodic electrode 22 therebetween. A window portion is formed on the other end side of the vacuum casing 21 at a position in a direction of an extension of the anodic electrode 22 and a light detecting portion 28 is attached to the other end side of the vacuum casing to abut thereon. A focusing lens 29 is attached to the other end side of the vacuum casing 21 so as to cover the window portion thereof and a plate 27 having a hole is attached to an inside of the vacuum casing 21 in a position near to the anodic electrode 22 from the other end side of the vacuum casing. The plate 27 having a hole is placed for preventing scattered particles due to sputter deposition from attaching to the focusing lens. A controlling unit, denoted by 30 and connected to a power source including a direct current power source 24 and an ammeter 25 and to a light detecting portion 28, conducts control of optical emission by discharge and calculation for analyzing gas based on a light intensity of discharge optical emission detected with the light detecting portion 28.

A hole is formed in the plate 27 having a hole at a position in a direction of extension of the anodic electrode 22, so that light emitted in a vicinity of the tip of the anodic electrode 22 passes through this hole as well as the focusing lens 29 to be directed towards the light detecting portion 28. While the vacuum casing 21, including the focusing lens 29 and its mounting portion, is tightly closable, the inside of the light detecting portion 28 may be under atmospheric pressure without necessity to be tightly closable. The light detecting portion 28 has an arrangement of multichannel spectrometer in which respective optical elements are disposed such that emitted light, after having passed through the focusing lens 29, passes through a hole of an aperture a, is reflected by a concave mirror b to be directed towards a diffraction grating c, and then diffracted light is reflected by a mirror d to arrive at a light receiving element e.

When the elongated ferromagnetic anodic electrode 22 is disposed at the center of a cylindrical magnet as magnetic field applying module 26, with the vacuum casing being of a cylindrical shape, a magnetic field M generated by the N-pole side of the magnetic field applying module 26 is concentrated in a vicinity of the tip of the elongated ferromagnetic anodic electrode 22. As the magnetic field M in a vicinity of tip of the ferromagnetic anodic electrode 22 is substantially perpendicular to an electric field E, electrons are localized in this area, so that intensive localized discharge optical emission is generated. As this discharge optical emission is intensive and localized, it can be focused on the light detecting module 28 in a high efficiency with the focusing lens 29, so that optical emission in a low partial pressure of gas of 10⁻⁷ Pa can be measured.

On the other hand, as the elongated ferromagnetic anodic electrode 22 has a character attracting a magnetic field, a magnetic field in an area on the S-pole side of the magnetic field applying module 26 becomes sparse and is not perpendicular to an electric field E. Due to this, discharge in an area of the S-pole side of the magnetic field applying module 26 is weak one to restrain sputter deposition in a vicinity of this area, thus having an effect of preventing the focusing lens from getting dirt by scattered particles.

Requirements of composition for a gas analyzer according to Example 1 with the vacuum casing 21 being of a cylindrical shape is as follows.

### (1a) Relation in dimensions of the casing

The inner diameter of the vacuum casing 21 is of 10 mm to 100 mm.

While discharge is generated with the vacuum casing 21 as a cathode electrode and the ferromagnetic anodic electrode 22 as an anode, discharge is not generated due to a shield effect in a case where a distance between the two electrodes is short. Here, as a result of preparing vacuum casings having various inner diameters and studying about stability of discharge, discharge is not generated with a vacuum casing having an inner diameter of 8 mm. On the other hand, while, with a vacuum casing having an inner diameter of 10 mm, discharge is stable in a high pressure of a medium vacuum, discharge is not stable in a low pressure of a high vacuum. From such a result, it can be said that an inner diameter of the vacuum casing 21 is preferable to be larger than 10 mm.

On the other hand, when the vacuum casing has a large inner diameter, concentration extent of magnetic field concentrated on the ferromagnetic anodic electrode 22 changes. The concentration extent of magnetic field depends on a magnetic force and a length of the magnet as magnetic field applying module 26 as well as permeability of the ferromagnetic material. As a result of simulation conducted on preset magnetic field, it can be said that an inner diameter of the vacuum casing 21 of 100 mm or less may be good and specifically an inner diameter of 50 mm or less is preferable.

### (1b) Ferromagnetic anodic electrode 22

(1b-1)
The ferromagnetic anodic electrode 22 serves as an electrode for applying a high voltage and has a role of concentrating a magnetic field M generated by a magnet as magnetic field applying module 26, and a ferromagnetic and soft magnetic material or a ferromagnetic and hard magnetic material is used for the electrode.

### (i) Soft magnetic material:

Soft magnetic metal materials having electric conductivity and a relative permeability of 2 or higher, such as iron, carbon steel or magnetic stainless steel are used. In a case where these material are used, discharge optical emission with a gas analyzer exhibited substantially same intensity. Further, as a result of having conducted magnetic field simulation in a gas analyzer with varied permeability of the ferromagnetic anodic electrode 22, magnetic field generated by the magnet as magnetic field applying module 26 was concentrated on the ferromagnetic anodic electrode 22 when the relative permeability is 2 or higher. From these results, it is preferable to use a conductive metal of soft magnetic material and having relative permeability of 2 or higher for the ferromagnetic anodic electrode 22 in a case of a soft magnetic material.

### (ii) Hard magnetic material: A permanent magnet having electric conductivity

On the other hand, a permanent magnet having electric conductivity may be used in a case where a permanent magnet of a hard magnetic material is used as the ferromagnetic anodic electrode 22, and it is preferable to use a neodymium magnet or a samarium-cobalt magnet as such a magnet. Here, in a case where a magnet of a hard magnet material is used for the ferromagnetic anodic electrode 22, a magnetic pole at a tip of the electrode is placed so as to concentrate a magnetic field generated by the magnet as magnetic field applying module 26.

### (1b-2) Position of a tip of the electrode

While the ferromagnetic anodic electrode 22 concentrates a magnetic field M generated by a magnet as magnetic field applying module 26, it is preferable here that the concentrated magnetic field is parallel to an axis of the electrode as well as the magnetic field has a high extent of concentration. Here, magnetic field simulation was conducted varying a position of a tip of the ferromagnetic anodic electrode 22 relative to the magnet as magnetic field applying module 26. As a result of this, a position of a tip of the ferromagnetic anodic electrode 22 may be anywhere from a position where the tip of the electrode protrudes by a half of a length of the magnet as magnetic field applying module 26 in an axial direction of the magnetic field to a position at an end of the magnet, and, further, it is desirable to position the tip of the electrode in a vicinity of the center of the magnet as magnetic field applying module 26.

### (1c) Direct current power source 24

As discharge occurs at a direct current voltage of several kV, a power source that can apply up to maximum voltage of 10 kV may be good.

### (1d) Magnetic field applying module 26

As the magnet as magnetic field applying module 26 is necessary for maintaining discharge in a pressure range over a high vacuum and more intensive magnetic force is preferable for it, its material is desirable to be a neodymium magnet or a samarium-cobalt magnet. On the other hand, while it has been taken as preferable in a case of conventional inverted magnetron magnetic field discharge method that an intensive magnet has a length in a direction of magnetic field axis of 5 mm or more, for example of 10 mm or more, in order to apply magnetic field parallel to an electrode axis in a vicinity of the anodic electrode, the length of a magnet as the magnetic field applying module 26 for discharge by the present invention may be shorter than the above so as to concentrate magnetic field on the ferromagnetic anodic electrode 22. Having measured intensity of discharge optical emission regarding some of magnets of various lengths, it was found that a length of a magnet of 5 mm or more may be good.

### (1e) Plate having 27 a hole

While material of the vacuum casing 21 is scattered due to sputter deposition by discharge, a plate 27 having a hole is placed in order to prevent the focusing lens 29 from getting dirt by this particles of sputter deposition. It is preferable for the position of the plate 27 having a hole to be apart from a tip of the anodic electrode 22 by the inner diameter or more of the vacuum casing. A material thereof may be either a metal or an insulator (dielectric) material. A material of a metal is subjected to sputter deposition by discharge, as the plate 27 having a hole serves as a cathode. In order to prevent this, the distance of the plate 27 having a hole from the tip of the ferromagnetic anodic electrode 22 is desirable to be longer than the inner radius of the vacuum casing 21. Further, in order to prevent abnormal discharge, a peripheral face of an opening of the hole in the plate 27 having a hole is desirable to have a smooth convex roundness.

In a case where the plate 27 having a hole is of an insulator material, the insulator material undergoes dielectric polarization when electric field is applied and a surface of the plate having a hole on the side where discharge occurs bears positive electricity, so that sputter deposition of the plate having a hole by discharge can be avoided. Consequently, a material of the plate 27 having a hole is preferable to be of an insulator material. In a case where a conductive material is used for a plate 27 having a hole, it is preferable to attach the plate 27 having a hole to the inside of the vacuum casing 21 via an insulator member.

### (1f) Focusing lens 29

The focusing lens 19 is desirable to be a quartz glass lens and its focusing length is determined taking a position of discharge optical emission and a position of the plate 27 having a hole into account. While the focusing lens 29 is placed so as to focus emitted light on the light receiving element e in the light detecting portion 28, it is required for the focusing lens 29 to transmit atomic optical emission and molecular optical emission of various gases as well as light of a measurable wave length range of the light detecting portion 28. In general, most of multichannel spectrometers used for the light detecting portion 28 are ones that detect light of 200 nm to 1000 nm. In this case, the focusing lens 29 is desirable to be a quartz glass lens transmitting ultraviolet light. Here, a lens made of silicate glass of a low cost may be used, as this transmits light of 400 nm or longer.

With a gas analyzer according to the present invention, a plate 27 having a hole is placed between light of discharge optical emission L and the focusing lens 29, so that light having passed through the hole in the plate having a hole is focused. While the focusing length is determined taking a position of optical emission by discharge and a position of the plate having a hole into account in this case, it may be preferable in general to adopt a lens having a focusing length by a half of the distance between the plate having a hole and the lens with a point light source at the position of the plate 27 having a hole.

### (1g) Light detecting portion 28

It is preferable to use a multichannel spectrometer. A multichannel spectrometer that can measure a plurality of wavelengths simultaneously in a high sensitivity is used for the light detecting portion 28. A multichannel spectrometer disperses incident light with a diffraction grating and detects the dispersed light with a CMOS sensor in which a multitude of CMOS (complementary metal oxide film semiconductors) are arrayed or a CCD sensor in which a multitude of CCD (charge coupled devices) are arrayed, thus conducting high sensitive detection of light for a plurality of wavelengths simultaneously.

### (1h) Controlling unit 30

The controlling unit 30, controlling the gas analyzer, has a function of controlling discharge in magnetic field and a function of converting signal of discharge optical emission into partial pressure. The gas analyzer according to the present invention controls discharge so that intensity of discharge optical emission of various gas molecules generally responds linearly to a total pressure and converts intensity of optical emission into a pressure of gas with intensity of intrinsic optical emission peculiar to gas species and pressure of gas (partial pressure) measured preliminarily.

### [Embodiment 2]

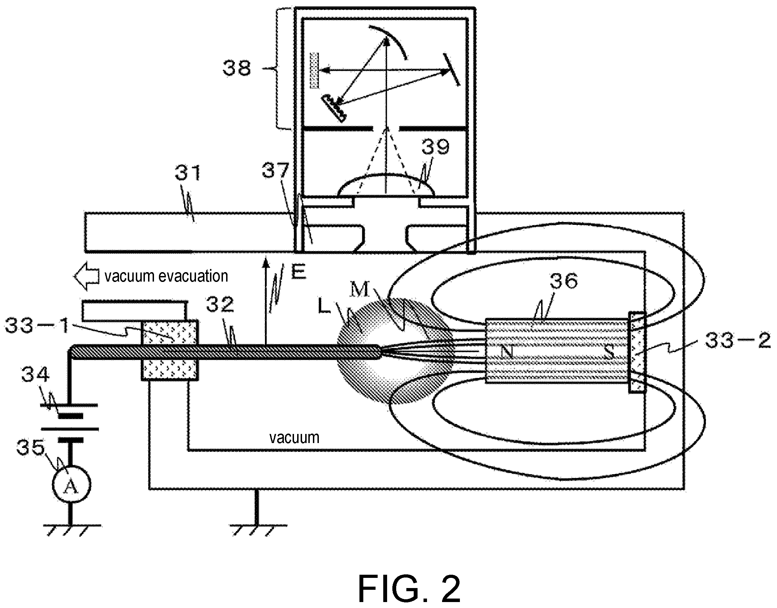

A gas analyzer according to Embodiment 2 shown in Fig. 2 is different from one according to Example 1 shown in Fig. 1 in having another arrangement of concentrating magnetic field M generated by a magnet as magnetic field applying module 36 on a tip of a thin ferromagnetic anodic electrode 32 and in that a light detecting portion 38 is attached to a different position, and similar to one according to Example 1 in the other ways.

In the gas analyzer shown in Fig. 2, the magnet as magnetic field applying module 36 is a thick bar magnet and is disposed to be opposed to an elongated ferromagnetic anodic electrode 32, in a direction of its extension, attached to one end face side of a tightly closable vacuum casing 31 via an insulator member 33-1 and extending in a longitudinal direction of the cylindrical vacuum case as a cantilever. Then, the bar magnet as magnetic field applying module 36 is attached to the other end face side of the cylindrical vacuum casing 31 via an insulator member 33-2 as a cantilever.

While magnetic field M is particularly concentrated between a tip of the ferromagnetic anodic electrode 32 and the magnetic pole opposed thereto of the bar magnet, so that localized discharge optical emission is generated in a vicinity of a tip of the anodic electrode when a high voltage is applied to the ferromagnetic anodic electrode 32, a light detecting portion 38 for detecting light of optical emission is provided on a peripheral side of the cylindrical vacuum casing 31. That is, the light detecting portion 38 is provided outside of the cylindrical casing so as to detect light advancing in one direction (upward direction in Fig.2) in a plane, passing a tip point of the ferromagnetic anodic electrode 32 and perpendicular to a longitudinal direction of the cylindrical casing. Here, a side part of the cylindrical casing 31 at a position where the light detecting portion 38 is attached is removed and a plate 37 having a hole for light of discharge optical emission to pass through is attached to fit into the position of this removed side part.

A focusing lens 39 is attached to the outside of the plate 37 having a hole via a plate for attaching a focusing lens and the light detecting portion 38 is attached thereto at a further outside position. Portions up to the plate for attaching a focusing lens and the focusing lens 39 along with the pipe-shaped vacuum casing 21 form a tightly closed composition and inside of the light detecting portion 38 may be in atmospheric pressure. It is preferable to use a multichannel spectrometer for the light detecting portion 38 similarly as Example 1.

As magnetic field M generated by the bar magnet as magnetic field applying module 36 is concentrated on a tip of the ferromagnetic anodic electrode 32 and perpendicular to an electric field E with the gas analyzer shown according to Embodiment 2 shown in Fig. 2, so that light of discharge optical emission L localized and enhanced in a vicinity of the tip of the ferromagnetic anodic electrode 32 can be attained. Here, in a case where the bar magnet as magnetic field applying module 36 is of a conductive material, it is desirable to insulate the bar magnet from the vacuum casing 31 as a cathode electrode, hence the bar magnet is attached to the other end face side of the vacuum casing 31 via an insulator member 33-2. On the other hand, the bar magnet as the magnetic field applying module 36 is of an insulator material, the bar magnet can be attached directly to the vacuum casing 31. In Fig. 2, 34 is direct current power source, and 35 is ammeter.

Requirements of composition for Embodiment 2 is as follows.

### (2a) Magnetic field applying module 36

A magnet that has a high Curie temperature without losing magnetic force even at a high temperature is suitable for the magnet as the magnetic field applying module, as the magnet is exposed to discharge at a high temperature. A samarium-cobalt magnet or a ferrite magnet is desired for such a magnet.

The other requirements for Embodiment 2 are similar to ones for Embodiment 1, and a controlling unit (not shown) that has a function of controlling discharge in magnetic field and a function of converting signal of discharge optical emission into partial pressure is provided similarly as Embodiment 1.

With Example 1 and Embodiment 2, a ferromagnetic electrode is placed in an area of discharge where Electric field E and magnetic field M are applied, and, by concentrating the magnetic field M generated by the magnet as magnetic field applying module on a tip of the elongated ferromagnetic anodic electrode, electrons are restrained to this area and light of discharge optical emission L is localized, thus increasing intensity of optical emission. In contrast to these two Embodiments, discharge optical emission can be further localized by composing a magnetic circuit for magnetic flux to be permeable well. Embodiments arranged as such will be explained below.

### [Embodiment 3]

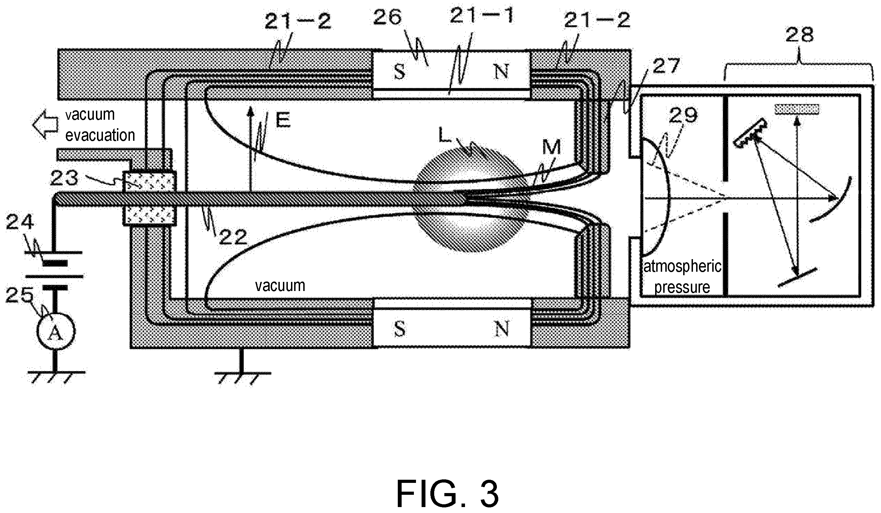

With a gas analyzer according to Embodiment 3 of the present invention shown in Fig. 3, an attaching portion of a magnet as magnetic field applying module 26 on a side face of a tightly closable cylindrical vacuum casing is a non-magnetic portion 21-1 of the vacuum casing and the other portion of the vacuum casing is a ferromagnetic potion 21-2 of the vacuum casing. Then, a magnetic circuit for magnetic flux to be permeable well is composed, by using a ferromagnetic plate 27 having a hole, from a magnet as magnetic field applying module 26 to a ferromagnetic anodic electrode 22 and from the magnet to the ferromagnetic plate 27 having a hole. With this, magnetic field M generated by the magnetic field applying module 26 and concentrated on the ferromagnetic anodic electrode 22 can be enhanced as well as cross electric field E perpendicularly and, along with this, electrons can be further restrained to this area, so that discharge can be localized and enhanced.

Requirements of composition for Embodiment 3 is as follows.

### (3a) Ferromagnetic portion 21-2 of vacuum casing

While the ferromagnetic portion 21-2 of the vacuum casing is of a ferromagnetic material considering of composing a magnetic circuit for magnetic flux to be permeable well, it is desirable to be of a ferromagnetic and soft magnetic material. Here, the non-magnetic portion 21-1 of the vacuum casing and the ferromagnetic portion 21-2 of the vacuum casing may be integrated causing both opposing ends to abut against each other thus joining both as through welding, or concave portions may be formed outside of the non-magnetic portion 21-1 with ferromagnetic sleeves placed on both sides of the magnet as magnetic field applying module 26 respectively.

### (3b) Ferromagnetic plate 37 having a hole

The plate 37 having a hole is of a ferromagnetic material in order to compose a magnetic circuit for magnetic flux to be permeable from the magnet as the magnetic field applying module 26 to the plate having a hole. Here, in order to prevent abnormal discharge and to generate magnetic field M effectively concentrated on the ferromagnetic anodic electrode 22, an inner peripheral face of an opening of the hole in the plate 27 having a hole is desirable to have a smooth convex roundness.

The other requirements for Embodiment 3 are similar to ones for Embodiment 1, and a controlling unit (not shown) that has a function of controlling discharge in magnetic field and a function of converting signal of optical emission by discharge into partial pressure is provided similarly as Example 1.

### [Embodiment 4, Embodiment 5]

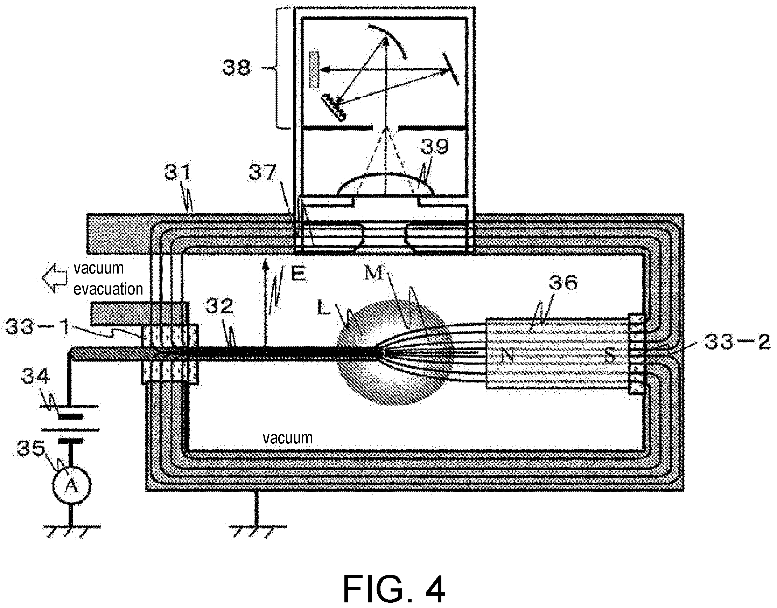

With a gas analyzers according to Embodiments 4 to 5, a magnetic circuit is formed for magnetic flux to be permeable well in an arrangement that provides a bar magnet as magnetic field applying module 26 as in Embodiment 2. The gas analyzer according to Embodiment 4 shown in Fig. 4 corresponds to one in which a vacuum casing 31 in Embodiment 2 is formed of a ferromagnetic and soft magnetic material. As a soft magnetic material causes magnetic flux to be permeable well, a magnetic circuit can be composed with an elongated ferromagnetic anodic electrode 32 - a ferromagnetic vacuum casing 31 - a bar magnet as magnetic field applying module 36, and magnetic field generated between the bar magnet as magnetic field applying module 36 and the elongated ferromagnetic anodic electrode 32 is enhanced. With the magnetic circuit composed for magnetic flux to be permeable well, discharge is further localized and intension of discharge optical emission L is increased as well as magnetic field substantially disappears in the other vacuum area, thus enabling sputter deposition to be restrained and preventing a focusing lens from getting dirt by scattered particles. In Fig. 4, 34 is direct current power source, and 35 is ammeter.

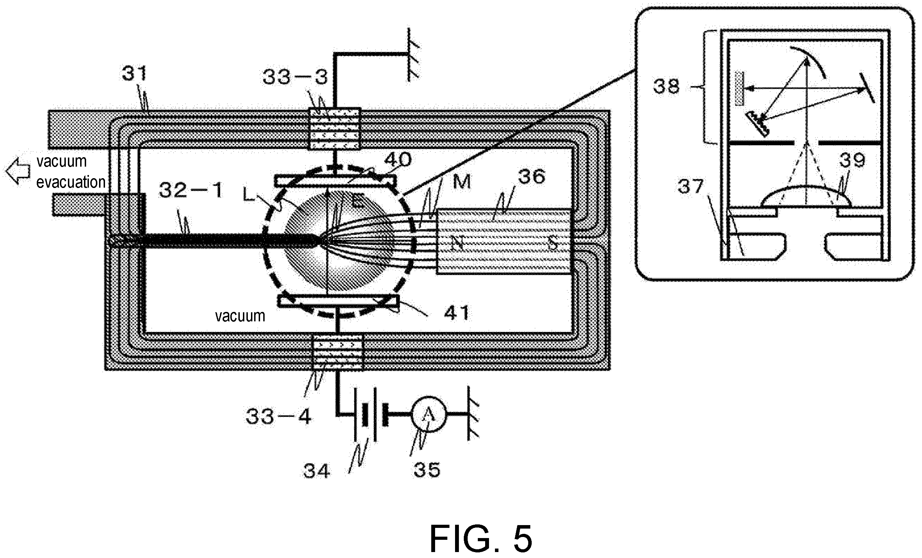

In a case where the ferromagnetic anodic electrode 32 and the thick bar magnet as the magnetic field applying module 36 are attached respectively via an insulator members 33-1, 33-2 in Fig. 4, magnetic loss occurs due to a low permeability of the insulator members. With a gas analyzer shown in Fig. 5 according to Embodiment 5, a ferromagnetic vacuum casing 31 is caused to be in floating, an elongated ferromagnetic member 32-1 attached to one end face side of a pipe-shaped vacuum casing 31 and extending as a cantilever and a bar magnet as magnetic field applying module 36 attached to the other end side of the vacuum casing are placed with tips thereof opposed to each other and connected to the vacuum casing to compose a perfectly closed magnetic circuit, thus causing magnetic loss to be extremely low. On the other hand, an anodic electrode 40 and a cathode electrode 41 are respectively provided in another way via insulator members 33-3, 33-4 in a manner to be opposed to each other with a tip of the elongated ferromagnetic member 32-1 disposed between the electrodes as well as insulated from the vacuum casing, so that electric field is generated at a position of a tip of the elongated ferromagnetic member 32-1.

Requirements of composition for Embodiments 4 to 5 are as follows.

### (4-5a) Ferromagnetic vacuum casing 31

While the ferromagnetic vacuum casing 31 is of a ferromagnetic material considering of composing a magnetic circuit for magnetic flux to be permeable well similarly as the ferromagnetic portion 21-2 of the vacuum casing in Embodiment 3, it is desirable to be of a ferromagnetic and soft magnetic material.

### (4-5b) Magnetic field applying module 36

A magnet that has a high Curie temperature without losing magnetic force even at a high temperature is suitable for a magnet in Embodiments 4 to 5, as the magnet is exposed to discharge at a high temperature similarly to a magnet in Embodiment 2, and a samarium-cobalt magnet or a ferrite magnet is desired for such magnet.

While a light detecting portion 38 is shown with a dotted-line in Embodiment 5 shown in Fig. 5, it is attached to the vacuum casing on a back side or front side as shown. The other requirements are similar to ones for Embodiment 2, and a controlling unit (not shown) that has a function of controlling discharge in magnetic field and a function of converting signal of optical emission by discharge into partial pressure is provided similarly as Example 1.

### [Composition of Electrode]

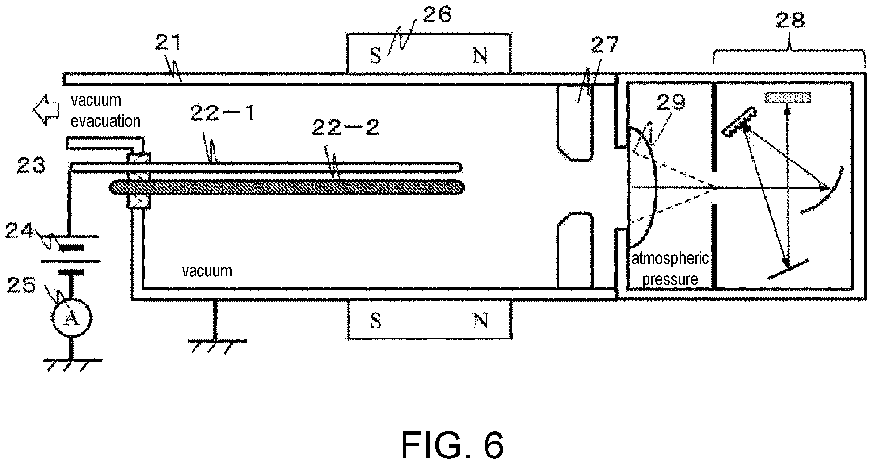

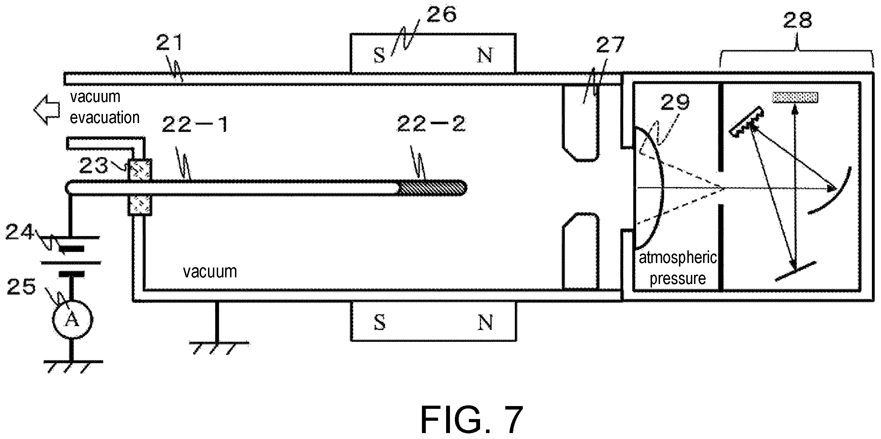

While magnetic field M generated by a magnet as magnetic field applying module is concentrated on a tip of a ferromagnetic anodic electrode by using a ferromagnetic material for the electrode in Example 1 and Embodiments 2 to 4, the ferromagnetic anodic electrode can take another form as shown in Figs. 6 to 7. Explanation will be made for another form of a ferromagnetic anodic electrode that corresponds to a replacement from one in Example 1, referring to Figs. 6 to 7. Also with a composition in which a non-magnetic anodic electrode 22-1 and a concurrently provided ferromagnetic material 22-2 are used as shown in Fig. 6 or a composition in which a tip of a non-magnetic anodic electrode 22-1 is formed to be of a ferromagnetic material 22-2 as shown in Fig. 7, magnetic field M generated by a magnet as magnetic field applying module 26 is effectively concentrated on a tip of the ferromagnetic material, so that localized and intensive discharge optical emission L can be attained.

### [Enhancement of discharge optical emission by introducing a tiny amount of gas]

With the present invention, it was found that discharge optical emission is enhanced when a tiny amount of gas other than one to be detected is introduced in detecting gas. While this utilizes a phenomenon such that, when ionized gas or excited gas having a long life collides with gas to be detected, the gas to be detected is excited, the phenomenon is different from a Penning ionization used in conventional lowering of voltage for discharging or enhancement of gas ionization.

Penning ionization is a phenomenon such that, when gas B having an ionization energy higher than an ionization energy of gas A desired to be ionized or having a metastable excitation energy near to the ionization energy is introduced into the gas A, the gas A is ionized with the gas B having been excited to a high energy state by discharge colliding with the gas A. In a discharging lamp, for example, mercury vapor is effectively ionized by introducing a tiny amount of argon gas, due to which breakdown voltage for discharge of the discharging lamp can be lowered. In film formation by sputter deposition, for another example, argon gas is effectively ionized by introducing a tiny amount of helium gas, due to which there is such an effect that sputter deposition by argon ions for forming film can be increased and speed of film formation can be raised.

With the present invention, a phenomenon was found such that, also when not only gas B having an ionization energy higher than an ionization energy of gas A desired to be ionized or having a high metastable excitation energy but also gas C having an ionization energy lower than the ionization energy of the gas A are introduced into the gas A, the gas A to be detected is excited and discharge optical emission is enhanced. This is because the gas A can be excited even using the gas C having a low ionization energy, as an excitation energy of the gas A for discharge optical emission is lower than an ionization energy or a high metastable excitation energy. That is, the introduced gas C is one having a metastable excitation energy higher than an excitation energy of the gas A for discharge optical emission.

In a case, for example, where the gas A to be detected was nitrogen gas and when helium gas (gas B) having an ionization energy higher than one of nitrogen gas was introduced, discharge optical emission of nitrogen gas was naturally enhanced. On the other hand, even when oxygen gas (gas C) having an ionization energy lower than one of nitrogen energy was introduced, discharge optical emission of nitrogen gas was enhanced. Besides, in a case where gas A to be detected was neon gas or argon gas and when helium gas (gas B) was introduced, discharge optical emission of these inert gases was naturally enhanced. On the other hand, even when nitrogen gas (gas C) having a low ionization energy was introduced, discharge optical emission of these inert gases was enhanced. The gases to be introduced in a tiny amount are desired to be helium gas, nitrogen gas, air gas and argon gas, taking safety, inertness and cost into consideration.

Here, with an amount of the introduced gas B or gas C being 1/100 to 1/10, discharge optical emission of gas to be detected was enhanced by two times to three times. Due to this, a lower limit of detection with the gas analyzer according to the present invention can be lowered.

In actual experiments, argon was used for gas to be detected. Helium gas, carbon dioxide gas, nitrogen gas and air were used for excitation assisting gases. An introduced amount of any of the excitation assisting gases was about 1/10 of the gas to be detected. As a result, optical emission of argon gas was enhanced by about 2 times to 2.5 times with any of the excitation assisting gases. First ionization energy of gaseous atom is shown in Table 1.

**[Table 1]**

| First ionization energy of gaseous atom | | |
|---|---|---|
| | kJ/mol | eV |
| H | 1312.0 | 13.59 |
| He | 2372.3 | 24.58 |
| C | 1086.5 | 11.26 |
| N | 1402.3 | 14.53 |
| O | 1313.9 | 13.61 |
| Ne | 2080.7 | 21.56 |
| Ar | 1520.6 | 15.75 |

### [Performance of gas analyzer according to the present invention]

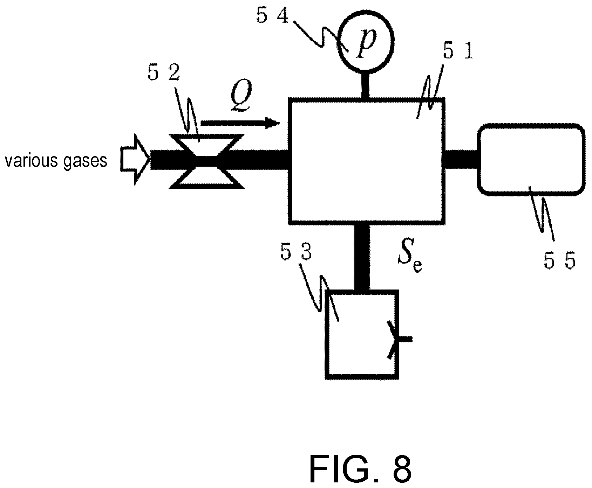

Performance of a gas analyzer according to the present invention was certified using a gas analyzer performance measuring apparatus shown in Fig. 8. With the gas analyzer performance measuring apparatus, a standard gas flow rate drawing-in equipment 52 is connected to a vacuum casing 51 and various gases with various flow rates Q [Pam³s⁻¹] were introduced into the vacuum casing 51. A high vacuum pump 53 with an effective gas exhausting speed at a gas intake port preset to be Se [m³s⁻¹] was connected to the vacuum casing 51. Then, a vacuum gauge 54 for measuring a total pressure and a gas analyzer 55 to be tested were connected respectively to the vacuum casing 51.

In test of a gas analyzer, causing a flow rate Q [Pam³s⁻¹] of gas to flow through, a pressure p [Pa] was obtained from a relation of Q = Se×p. On the other hand, an intrinsic optical mission of gas introduced then into the gas analyzer was measured. Helium, nitrogen, oxygen, argon and carbon dioxide, as typical gases, were used for gas species and flow rate Q were set to be varied sequentially in a range of 10⁻⁹ to 10⁻⁴ Pam³s⁻¹. Here, a standard conductance element according to a national secondary standard was used as the gas flow rate drawing-in equipment 52.

In order to demonstrate performance of a gas analyzer using discharge optical emission according to the present invention, performance of the following four gas analyzers were compared respectively, in which various gases were introduced using a gas analyzing-evaluating equipment shown in Fig. 8:
(1) a gas analyzer corresponding to Fig. 9 that has an electrode of a conventional non-magnetic electrode (referred to as a conventional gas analyzer 1),
(2) a gas analyzer corresponding to Fig. 11 that has an electrode of a conventional non-magnetic electrode and has two cylindrical magnets provided (referred to as a conventional gas analyzer 2),
(3) a gas analyzer according to Example 1 of the present invention that has an electrode of a magnetic material, and
(4) a gas analyzer according to Embodiment 3 of the present invention with a magnetic circuit composed.

Here, with the gas analyzer according to Embodiment 3, sleeves of a ferromagnetic material according to Embodiment 1 were provided on both sides of the magnet as magnetic field applying module as well as a plate of a ferromagnetic material having a hole was placed in the gas analyzer. The results are shown in Table 2.

**[Table 2]**

| Performance comparison among the gas analyzer according to the present invention and conventional gas analyzer | | | | |
|---|---|---|---|---|
| | Conventional gas analyzer 1 | Conventional gas analyzer 2 | Gas analyzer according to the present invention Example 1 1 | Gas analyzer according to the present invention Embodiment 3 |
| Pressure range of discharge | 10⁻⁶ Pa to 10² Pa | 10⁻⁷ Pa to 10² Pa | 10⁻⁷ Pa to 10² Pa | 10⁻⁷ Pa to 10² Pa |
| Lower limit of detection of partial pressure | 10⁻⁵ Pa | 10⁻⁵ Pa | 10⁻⁷ Pa 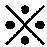 5×10⁻⁸ Pa when a tiny amount of gas was introduced. | 5×10⁻⁸ Pa 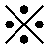 3×10⁻⁸ Pa when a tiny amount of gas was introduced. |
| Linearity of sensitivity | Not so good at 10⁻² Pa or more | Not so good at 10⁻² Pa or more | Good 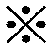 Not so good at 10 Pa or more. | Good 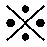 Not so good at 10 Pa or more. |
| Durability (Dirt prevention performance) | Good | Good | Very good | Very good |

By introducing a tiny amount of gas other than a gas to be detected, in detecting gas, discharge optical emission was enhanced by several times and a lower limit of detection of partial pressure was lowered further with the present invention, so these results are also shown in the Table 2. Gas analyzers according to Example 1 and Embodiment 3 of the present invention can upgrade a lower limit of detection of partial pressure by 2 digits or more, is excellent in linearity of sensitivity and improves durability.

Here, it was also certified that the gas analyzer according to Embodiment 2 of the present invention exhibits similar performance as one according to Example 1 and the gas analyzer according to Embodiments 4 to 5 exhibits similar performance as Embodiment 3.

### [Application to various vacuum equipment]

As the gas analyzer according to the present invention was applied to a film forming apparatus by sputter deposition and a leakage inspection apparatus and then operated, argon gas as process gas, in the first, and remaining gases such as water vapor were detected. Further, with a practical leakage inspection apparatus, it was possible to detect a leakage flow rate of 10⁻⁸ Pam³s⁻¹ within a practical short machine time of 60 sec.

### [Applicability in Industry]

The present invention is applicable to gas analyzing using various vacuum equipment such as (1) a vacuum equipment for manufacturing devices, (2) a leakage inspection apparatus and (3) a vacuum melting furnace, a vacuum freezing-drying apparatus.

### [Notation of Reference number]

- 1: vacuum casing
- 2: electrode
- 3: insulated terminal
- 4: direct current power source
- 5: ammeter
- 6: magnetic field applying module
- 7: plate having a hole
- 8: focusing lens
- 9: light detecting module
- 21: vacuum casing
- 21-1: non-magnetic portion of vacuum casing
- 21-2: ferromagnetic portion of vacuum casing
- 22: ferromagnetic anodic electrode
- 22-1: non-magnetic anodic electrode
- 22-2: ferromagnetic member
- 23: insulator member
- 24: direct current power source
- 25: ammeter
- 26: magnetic field applying module
- 27: plate having a hole
- 28: light detecting portion
- 29: focusing lens
- 30: controlling unit
- 31: vacuum casing
- 32: ferromagnetic anodic electrode
- 32-1: ferromagnetic member
- 33-1: insulator member
- 33-2: insulator member
- 33-3: insulator member
- 33-4: insulator member
- 34: direct current power source
- 35: ammeter
- 36: magnetic field applying module
- 37: plate having a hole
- 38: light detecting portion
- 39: focusing lens
- 40: anodic electrode
- 41: cathode electrode
- 51: vacuum casing
- 52: standard gas flow-rate drawing-in equipment
- 53: high vacuum pump
- 54: vacuum gauge
- 55: gas analyzer to be tested
- E: electric field
- L: light of discharge optical emission
- M: magnetic field
- a: aperture
- b: concave mirror
- c: diffraction grating
- d: mirror
- e: light receiving element

## Claims

1. A gas analyzer using discharge optical emission that comprises a tightly closable vacuum casing (21, 31) formed to have a generally pipe shape, an anodic electrode (22, 32) and a cathode electrode provided in the vacuum casing (21, 31), magnetic field applying module (26, 36) generating magnetic field (M) in a direction crossing electric field (E) generated when a high voltage is applied between the anodic electrode (22, 32) and the cathode electrode, and light detecting module (28, 38) for detecting discharge optical emission (L) generated in magnetic field (M) when a high voltage is applied between the anodic electrode (22, 32) and the cathode electrode, wherein the anodic electrode (22, 32), the cathode electrode and/or the magnetic field applying module (26, 36) is/are composed with material, configuration and/or arrangement thereof adjusted so as to concentrate and/or enhance magnetic field (M) generated in an area of discharge optical emission when a high voltage is applied between the anodic electrode (22, 32) and the cathode electrode and thus to localize as well as enhance discharge optical emission (L), enabling a lower limit of detection to be attained,
wherein the vacuum casing (21, 31) is formed to be made of a conductive material so as to serve as the cathode electrode, the anodic electrode (22, 32) is made up of an elongated conductive member fixed to the vacuum casing (21, 31) on one end face side thereof so as to extend in a longitudinal direction within the vacuum casing (21, 31) as a cantilever,
wherein the gas analyzer is **characterized in that** at least a tip side portion of the anodic electrode (22, 32) is formed of a conductive ferromagnetic material, and a magnet as magnetic field applying module (26, 36) is placed in an area of discharge optical emission between the anodic electrode (22, 32) and an inner peripheral portion of the vacuum casing (21, 31) serving as the cathode electrode so as to concentrate magnetic field (M), so that discharge optical emission (L) is localized in a vicinity of a tip of the anodic electrode (22, 32).

2. The gas analyzer according to claim 1,
wherein the magnet as the magnetic field applying module (26) is provided on a peripheral face of the vacuum casing (21) surrounding a position of the tip of the anodic electrode (22), so that discharge optical emission (L) is localized in a vicinity of the tip of the anodic electrode (22).

3. The gas analyzer according to claim 1,
wherein the anodic electrode (32) is fixed to the vacuum casing (31) on one end face side thereof and, along therewith, a bar magnet as a bar magnetic field applying module (36) is fixed to the vacuum casing (31) on the other end face side so as to extend as a cantilever in a direction of extension of the anodic electrode (32), and a tip of the anodic electrode (32) of the ferromagnetic material and one pole of the bar magnet are in an opposed positional relation with each other to concentrate magnetic field (M) in a vicinity of the tip of the anodic electrode (32), so that discharge optical emission (L) is localized in a vicinity of the tip of the anodic electrode (32).

## Patentansprüche

1. Gasanalysator, der optische Entladungsemission nutzt, umfassend ein dicht verschließbares Vakuumgehäuse (21, 31), das im Wesentlichen rohrförmig ausgebildet ist, eine Anodenelektrode (22, 32) und eine Kathodenelektrode, angeordnet in dem Vakuumgehäuse (21, 31), ein Magnetfeld-Anlegemodul (26, 36), das ein Magnetfeld (M) in einer Richtung erzeugt, die das elektrische Feld (E) kreuzt, das erzeugt wird, wenn eine Hochspannung zwischen der Anodenelektrode (22, 32) und der Kathodenelektrode angelegt wird, und ein Lichtdetektionsmodul (28, 38) zum Detektieren der optischen Entladungsemission (L), die in dem Magnetfeld (M) erzeugt wird, wenn eine Hochspannung zwischen der Anodenelektrode (22, 32) und der Kathodenelektrode angelegt wird, wobei die Anodenelektrode (22, 32), die Kathodenelektrode und / oder das Magnetfeld-Anlegemodul (26, 36) aus Material, Konfiguration und / oder deren Anordnung gebildet sind, die so angepasst sind, dass das Magnetfeld (M), das in einem Bereich der optischen Entladungsemission erzeugt wird, wenn eine Hochspannung zwischen der Anodenelektrode (22, 32) und der Kathodenelektrode angelegt wird, konzentriert wird, um die optische Entladungsemission (L) zu lokalisieren und zu verstärken, wodurch eine niedrigere Nachweisgrenze erreicht werden kann,
wobei das Vakuumgehäuse (21, 31) so ausgebildet ist, dass es aus einem leitfähigen Material gebildet werden kann, um als Kathodenelektrode zu dienen, wobei die Anodenelektrode (22, 32) aus einem länglichen leitfähigen Element gebildet ist, das an einer Stirnseite des Vakuumgehäuses (21, 31) befestigt ist, sodass es sich in einer Längsrichtung innerhalb des Vakuumgehäuses (21, 31) als Ausleger erstreckt,
wobei der Gasanalysator **dadurch gekennzeichnet ist, dass**
zumindest ein spitzenseitiger Abschnitt der Anodenelektrode (22, 32) aus einem leitfähigen ferromagnetischen Material gebildet ist, und ein Magnet als Magnetfeld-Anlegemodul (26, 36) in einem Bereich der optischen Entladungsemission zwischen der Anodenelektrode (22, 32) und einem inneren Umfangsbereich des Vakuumgehäuses (21, 31), der als Kathodenelektrode dient, angeordnet ist, um das Magnetfeld (M) zu konzentrieren, sodass die optische Entladungsemission (L) in der Nähe der Spitze der Anodenelektrode (22, 32) lokalisiert ist.

2. Gasanalysator nach Anspruch 1,
wobei der Magnet als Magnetfeld-Anlegemodul (26) an einer Umfangsfläche des Vakuumgehäuses (21) angeordnet ist, die eine Position der Spitze der Anodenelektrode (22) umgibt, sodass die optische Entladungsemission (L) in der Nähe der Spitze der Anodenelektrode (22) lokalisiert ist.

3. Gasanalysator nach Anspruch 1,
wobei die Anodenelektrode (32) an einer Stirnseite des Vakuumgehäuses (31) befestigt ist und entlang dieser ein Stabmagnet als Stabmagnetfeld-Anlegemodul (36) an der anderen Stirnseite des Vakuumgehäuses (31) befestigt ist, sodass er sich als Ausleger in einer Erstreckungsrichtung der Anodenelektrode (32) erstreckt, und eine Spitze der Anodenelektrode (32) aus dem ferromagnetischen Material und ein Pol des Stabmagneten einander gegenüberliegen, um das Magnetfeld (M) in der Nähe der Spitze der Anodenelektrode (32) zu konzentrieren, sodass die optische Entladungsemission (L) in der Nähe der Spitze der Anodenelektrode (32) lokalisiert ist.

## Revendications

1. Analyseur de gaz utilisant l'émission optique par décharge, comprenant
une enceinte sous vide (21, 31) pouvant être fermée hermétiquement et présentant une forme généralement tubulaire, une électrode anodique (22, 32) et
une électrode cathodique disposées dans l'enceinte sous vide (21, 31),
un module d'application de champ magnétique (26, 36) générant un champ magnétique (M) dans une direction croisant le champ électrique (E) généré lorsqu'une haute tension est appliquée entre l'électrode anodique (22, 32) et l'électrode cathodique, et
un module de détection de lumière (28, 38) destiné à détecter l'émission optique de décharge (L) générée dans le champ magnétique (M) lorsqu'une haute tension est appliquée entre l'électrode anodique (22, 32) et l'électrode cathodique, dans lequel l'électrode anodique (22, 32), l'électrode cathodique et/ou le module d'application de champ magnétique (26, 36) sont composés d'un matériau, d'une configuration et/ou d'un agencement ajustés de manière à concentrer et/ou à renforcer le champ magnétique (M) généré dans une zone d'émission optique de décharge lorsqu'une haute tension est appliquée entre l'électrode anodique (22, 32) et l'électrode cathodique, et ainsi de localiser et d'amplifier l'émission optique de décharge (L), permettant d'atteindre une limite inférieure de détection,
dans lequel l'enceinte sous vide sous vide (21, 31) est formée d'un matériau conducteur de manière à servir d'électrode cathodique,
l'électrode anodique (22, 32) est constituée d'un élément conducteur allongé fixé à l'enceinte sous vide (21, 31) sur l'une de ses faces d'extrémité de manière à s'étendre dans une direction longitudinale à l'intérieur de l'enceinte sous vide (21, 31) en porte-à-faux, dans lequel l'analyseur de gaz est **caractérisé en ce que** au moins une partie du côté de la pointe d'électrode anodique (22, 32) est formée d'un matériau ferromagnétique conducteur, et
un aimant servant de module d'application de champ magnétique (26, 36) est placé dans une zone d'émission optique de décharge entre l'électrode anodique (22, 32) et
une partie périphérique interne de l'enceinte sous vide (21, 31) servant d'électrode cathodique de manière à concentrer le champ magnétique (M), de sorte que l'émission optique de décharge (L) soit localisée à proximité d'une pointe de l'électrode anodique (22, 32).

2. Analyseur de gaz selon la revendication 1, dans lequel l'aimant servant de module d'application de champ magnétique (26) est prévu sur une face périphérique de l'enceinte sous vide (21) entourant une position de la pointe de l'électrode anodique (22), de sorte que l'émission optique de décharge (L) est localisée à proximité de la pointe de l'électrode anodique (22).

3. Analyseur de gaz selon la revendication 1, dans lequel l'électrode anodique (32) est fixée à l'enceinte sous vide (31) sur l'une de ses faces d'extrémité et, parallèlement à celle-ci, un aimant en barre servant de module d'application de champ magnétique en barre (36) est fixé à l'enceinte sous vide (31) sur l'autre face d'extrémité de manière à s'étendre en porte-à-faux dans la direction d'extension de l'électrode anodique (32), et
une pointe de l'électrode anodique (32) en matériau ferromagnétique et un pôle de l'aimant en barre sont en relation de position opposée l'un par rapport à l'autre afin de concentrer le champ magnétique (M) à proximité de la pointe de l'électrode anodique (32), de sorte que l'émission optique de décharge (L) soit localisée à proximité de la pointe de l'électrode anodique (32).
